# EUROPEAN PATENT SPECIFICATION

(11) **EP 3 995 127 B1**
(45) Date of publication and mention of the grant of the patent: **25.02.2026**
(21) Application number: 20206263.4
(22) Date of filing: 06.11.2020
(51) Int. Cl.: A61K 8/44, A61K 8/49, A61K 8/60, A61Q 11/00, A61Q 19/00

(54) **SOLUBILIZER FREE FROM ETHOXYLATED EMULSIFIERS**
LÖSUNGSVERMITTLER OHNE ETHOXYLIERTE EMULGATOREN
SOLUBILISANT SANS EMULSIFIANTS ETHOXYLÉS

(43) Date of publication of application: 11.05.2022
(73) Proprietor: BASF SE, 67056 Ludwigshafen (DE)
(72) Inventor: Kempers, Peter, 40589 Düsseldorf (DE); Hloucha, Matthias, 40589 Düsseldorf (DE); Seidler, Stefanie, 40589 Düsseldorf (DE); Hackel, Stefanie, 40589 Düsseldorf (DE); Rinklieb, Ronny, 40589 Düsseldorf (DE)
(74) Representative: BASF IP Association

(56) References cited:
- EP-A1- 2 335 681
- EP-A2- 1 502 644
- US-A1- 2019 365 623

## Description

### Field of the invention

The present invention relates to a solubilizer composition especially for the cosmetics and home care industry which, although free from ethoxylated surfactants, has improved solubilizing capacity. It comprises a combination of alkylpolyglucosides, acylglutamic acid compound and sorbitan fatty acid esters.

### Background of the invention

Lipophilic substances such as vitamins, perfume oils or UV light protection filters are often difficult to incorporate into cosmetic, pharmaceutical, personal or home care preparations, especially if the final products have a high water content, so that in the manufacture of homogeneous pharmaceutical or cosmetic preparations the solubilization of hydrophobic substances has gained very great practical significance.

Solubilization is to be understood as meaning an improvement in solubility of a lipophilic substance in an aqueous matrix achieved by surface-active compounds which are able to convert sparingly water-soluble or water-insoluble substances into clear, at most opalescent aqueous solutions, without a change of the chemical structure of these substances in the process. The produced solubilisates are characterized in that the poorly water-soluble or water-insoluble substances are present in dissolved form by building molecular associates with surface-active compounds, the so-called micelles, in the aqueous solution. The resulting solutions are stable single-phase systems, that appear optically clear to opalescent and can be produced without great input of energy. Solubilizers also improve the appearance of cosmetic and personal care formulations and of food preparations, by making the formulations transparent. In case of pharmaceutical preparations solubilization can also influence the bioavailability and thus the effect of drugs.

On the personal care and home care market there is a large variety of different solubilizer types that are based on ethylenoxide/propylenoxide (EO/PO). EO/PO based solubilizer show a superior solubilization performance for different applications. "EO" denotes a structural unit derived from ethylene oxide -CH2CH2O-, "PO" analogous the structural unit derived from propylene oxide, i.e. -CH2CH2CH2O. As solubilizers for pharmaceutical drugs and cosmetic active ingredients mainly ethoxylated (hydrogenated) castor oil, (e.g. Cremophor^{®} brand, Eumulgin^{®} CO 40, Fa. BASF), ethoxylated sorbitan fatty acid esters (e.g. Tween^{®} marks, Fa. ICI) or ethoxylated hydroxystearic acid, (e.g. Solutol^{®} marks, Fa. BASF) are used as the solubilizing products.

For example EP 2 008 708 A2 describes such kind of solubilizer with ethoxylated fatty alcohol components as improved solubilizer. Although these known solubilizers have proven successful, there is still a need of further improvement combining a decreased eco-toxicology with good solubilization and application properties, and also physiological compatibility. The above-described, ethoxylated, previously used solubilizers exhibit a number of application technical disadvantages.

In order to avoid a harmful penetration through the skin barrier many customers prefer non-EO based products to enable more natural and eco-conform solutions and products. In the cosmetic, pharmaceutical, food and personal care area odor, appearance and taste is of great importance. Often fragrances, perfume oils and flavoring agents, which are usually lipophilic and have poor water solubility must be incorporated to achieve transparent aqueous compositions with pleasant odor or taste, so that the used solubilizer should not have any odor or taste themselves and should neither influence or mask the scent of the incorporated fragrance.

Alternatives for ethoxylated solubilizers often include mixtures comprising non-ionic surfactants, especially to fulfill the need of providing raw materials from renewable sources. From European application EP1 211 258 A1 it is known that mixtures of alkylpolyglucosides are effective solubilizers. Often these are combined with other ecologically - friendly nonionic surfactants which are based on renewable raw materials to improve solubilizing capacity.

EP 1 502 644 A2 relates to ethoxylate-free emulsifier combinations useful in the preparation of nanoemulsions and in perfume oil solubilization, comprising an alkyloligoglycoside, a polyolpolyhydroxystearate and an acyl glutamate. Preferably, the combination comprises, in addition to those surfactants, sorbitan stearate.

EP 2 366 376 A1 discloses solubilizing compositions comprising a mixture of specific alkylated glucosides, acylated amino acids, polyglycerol esters and C6 to C10 fatty acid monoglycerides. However, not only the choice of the different surfactants in the system is responsible for the solubilizing properties, also the amounts of each component have an influence on the improvement of solubilization. There is still a need for easily processable and precisely assembled systems to solubilize specific groups of molecules with low water solubility without an impact on odor, taste and color of formulations. It is an object of the present invention to provide novel solubilizer compositions which combine improved solubilization properties, improved stability, good physiological compatibility and dissolve perfume oils, fragrances, and essential oils with high clarity and long stability of scent, taste and appearance.

### Description of the invention

The problem stated above has been solved by the solubilizer composition, which is free from ethoxylated emulsifiers comprising
a) 20 to 40 wt % of alkyl glucosides,
b) 15 to 30 wt % of acyl glutamic acid compounds,
c) 2 to 6 wt % of a sorbitan fatty acid ester and
optionally 10 to 63 wt % of water, all wt % based on the weight of the composition and amounts given in wt % of active matter.

This solubilizer composition is an EO/PO-free mixture of solubilizers of natural origin optimized to easily incorporate perfume oils, fragrances, essential oils, dyes, flavorings, nutrients or vitamins in various aqueous formulations. It enables the clear solubilization especially of perfume oils, fragrances, and essential oils as the mixture is almost odorless itself, so that the smells of the incorporated odorous substances perfectly come to their own scent within the final product. The solubilizer composition is electrolyte-resistant and works in a very broad pH-range, also below 7, which is especially advantageous for skin tolerance of cosmetic compositions. Products comprising the solubilizer composition show a long-term stability in respect of appearance especially transparency and odor.

More preferably the solubilizer composition, which is free from ethoxylated emulsifiers is comprising
a) 25 to 35 wt % of alkyl glucosides,
b) 17 to 25 wt % of acyl glutamic acid compounds,
c) 3 to 5 wt % of a sorbitan fatty acid ester and

optionally 20 to 55 wt % of water, all wt % based on the weight of the composition and most preferably the solubilizer composition, which is free from ethoxylated emulsifiers is comprising
   a) 27 to 29 wt % of alkyl glucosides,
   b) 19 to 22 wt % of acyl glutamic acid compounds,
   c) 3.5 to 4 wt % of a sorbitan fatty acid ester and
optionally 30 to 50 wt % of water, and
optionally a pH-regulator and/or preservative, all wt % based on the weight of the composition and ranges given in wt % active matter.

Specifically the solubilizer composition which is free from ethoxylated emulsifiers is comprising
a) 27 to 29 wt % of alkyl glucosides,
b) 19 to 22 wt % of acyl glutamic acid compounds,
c) 3.5 to 4 wt % of a sorbitan fatty acid ester,
d) a pH-regulator and/or preservative and
e) 40 to 45 wt % of water, all wt % based on the weight of the composition and ranges given in wt % of active matter.

The inventive EO free solubilizer compound based on alkyl glucosides, acylglutamates and sorbitan fatty acid esters showed a similar solubilization performance to ethoxylated solubilizers and improved solubilizing properties compared to the mixture of alkylated glucosides, acylated amino acids, polyglycerol esters and C6 to C10 fatty acid monoglycerides. It could be shown that especially the amount of sorbitan fatty acid ester is relevant for the solubilization result.

The solubilizer composition according to the invention may be used in the form of a concentrate of all the above-mentioned solubilizer components but can as well be diluted since it is meant to be used in aqueous compositions after dissolving the sparingly water-soluble components. Solubilization of sparingly water-soluble components usually takes place by dissolving or dispersing the component in the solubilizer composition and afterwards mixing this solution with water. The dissolved component does not precipitate during the dilution with water and during storage of the diluted product.

In specific embodiments, within the framework of present claim 1, the inventive solubilizer composition could as well be defined by molar ratios as a solubilizer composition, which is free from ethoxylated emulsifiers comprising
a) alkyl glucosides,
b) acyl glutamic acid compounds,
c) sorbitan fatty acid ester and
d) water
characterized in that the ratio of a) to b) to c) is (6 to 8) to (4 to 6) to (1 to 2), preferably the ratio between a), b) and c) is (6.5 to 7.5) to (4.5 to 5.5) to (0.8 to 1.2).

As the amount of water can vary, this definition would as well be valid for the concentrated solubilizer composition as for the diluted composition.

### Component (A)

The compounds (A), which are referred to in the context of the present invention as alkyl glucosides, are obligatory for the solubilizer compositions according to the invention. They preferably have the following formula (I),

R¹O-[G]ₚ (I)

in which R¹ is an alkyl and/or alkenyl radical with 8 to 18 carbon atoms, G is glucose and p is numbers between 1 and 10. With regard to the compounds (A), the proviso also applies that the fraction of the compounds (A) in which the radical R¹ is an alkyl or alkenyl radical with 15 or more carbon atoms - based on the total amount of the compounds (A) in the aqueous surfactant compositions - is 5% by weight or less.

It may expressly be stated that the naming of the compounds (A) as alkyl glycosides - henceforth also referred to as APGs (singular: APG) - serves merely for a linguistically simple naming of the compounds (A) and should not be understood as being structurally limiting; hence in the definition according to the formula of the compounds (A) it is clarified that the radical R¹ can mean either an alkyl or an alkenyl radical and also - as the index p shows - that they can be alkyl or alkenyl oligoglucosides.

APGs of the form claimed here can be obtained by the relevant methods of preparative organic chemistry. The APGs are derived from glucose.

The index number p in the general formula (I) indicates the degree of oligomerization (DP degree = degree of polymerization). The degree of oligomerization of the APGs is between 1 and 10 and preferably between 1 and 6. Whereas p in an individual APG molecule must always be an integer and here in particular assumes the values in the range from 1 to 6, the value p for an APG which is a mixture of different APG molecules, which differ in their individual p values, is an analytically determined calculated parameter which in most cases is a fraction. Preferably, APGs are used with an average degree of oligomerization p in the range from 1.1 to 3.0. In this connection, preference is given in particular to those APGs whose average degree of oligomerization is less than 2 and is preferably in the range from 1.1 to 1.8 and in particular in the range from 1.2 to 1.7.

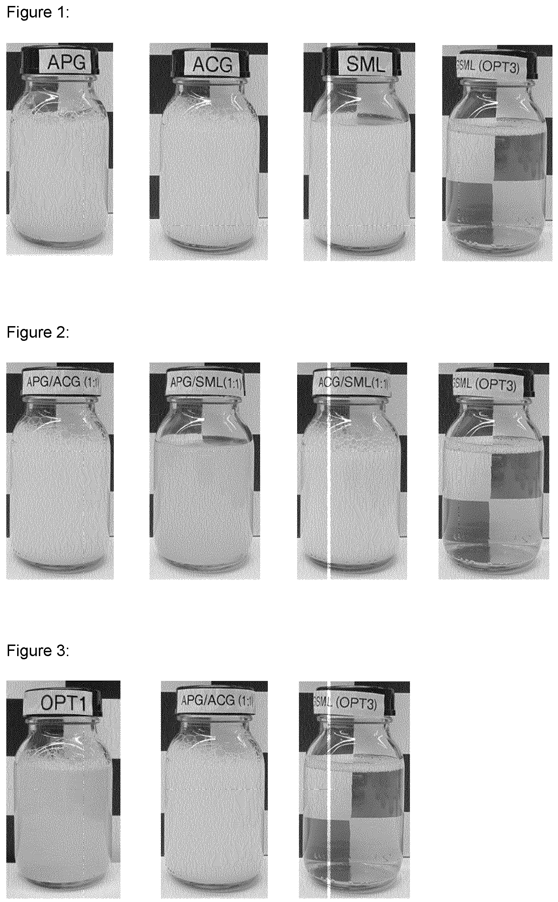

The average degree of oligomerization here is to be understood in the sense of how it is defined in the monograph K. Hill, W. von Rybinski, G. Stoll "Alkyl Polyglycosides. Technology, Properties and Applications" (VCH-Verlagsgesellschft, 1996) in the section "Degree of polymerization" (compare pages 11-12 of the book): there it reads "The average number of glycose units linked to an alcohol group is described as the (average) degree of polymerization (DP)." In explanatory figure 2, which describes a typical distribution of dodecyl glycoside oligomers of an AOPG with a degree of DP of 1.3, the average degree of DP is also described by a corresponding mathematical formula.

The radical R¹ is preferably derived from primary alcohols with 4 to 12 carbon atoms and preferably 8 to 10 carbon atoms. Typical examples of suitable radicals R¹ are butyl, hexyl, octyl, decyl, undecyl, dodecyl and myristyl. They are derived from the saturated fatty alcohols butanol-1, caproic alcohol (hexanol-1), caprylic alcohol (octanol-1), capric alcohol (decanol-1), undecanol-1, lauryl alcohol (dodecanol-1) and myristyl alcohol (tetradecanol-1), as are obtained for example in the hydrogenation of technical-grade fatty acid methyl esters or in the course of the hydrogenation of aldehydes during Roelen oxo synthesis.

Preference is given to APGs which are derived from glucose and in which the radical R¹ is a saturated alkyl radical with 8 to 12 carbon atoms and which have an average degree of oligomerization in the range from 1.1 to 3 and in particular in the range from 1.2 to 1.8 and particularly preferably in the range from 1.2 to 1.7. These APGs can for example be prepared by reacting glucose under acid catalysis with a fatty alcohol mixture, the fatty acid mixture used preferably being a forerunning produced during the distillative separation of technical-grade C₈₋₁₈-coconut fatty alcohol, which comprises predominantly octanol-1 and decanol-1 and also small amounts of dodecanol-1.

### Component (B)

The compounds (B), which are referred to in the context of the present invention as **N-acylglutamic acid compounds,** are obligatory for the solubilizer compositions according to the invention. They have in particular the following formula (II)

M¹OOC-CH₂-CH₂-CH(NH-CO-R²)-COOM² (II)

in which the radical R² is a linear or branched alkyl or alkenyl radical with 7 to 19 carbon atoms and the radicals M¹ and M² - independently of one another - are selected from the group H, Li, Na, K, Ca/2, Mg/2, ammonium and alkanolamines. In this connection, particularly preferred alkanolamines are monoethanolamine, diethanolamine, triethanolamine and monoisopropanolamine. In one embodiment, the radicals M¹ and M² are both Na (sodium).

In one embodiment, the proviso applies that the fraction of the compounds (B) in which the radical R² is an alkenyl radical - based on the total amount of the compounds (B) in the aqueous surfactant compositions - is 3% by weight or less.

The compounds (B) can be prepared by all of the methods known appropriately to the person skilled in the art.

The preferred N-acylglutamic acid compounds is sodium cocoyl glutamate

### Component (C)

The compounds (C), which are referred to in the context of the present invention as **sorbitan fatty acid esters,** are obligatory for the solubilizer compositions according to the invention.

Suitable sorbitan esters are selected from the group consisting of sorbitan monolaurate, sorbitan monopalmitate, sorbitan monostearate, sorbitan tristearate, sorbitan monooleate, sorbitan trioleate sorbitan sesquioleate, sorbitan monoisostearate, sorbitan sesquiisostearate, sorbitan diisostearate, sorbitan triisostearate, sorbitan dioleate, sorbitan mono myristate, sorbitan mono palmitate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and mixtures thereof.

The sorbitan fatty acid ester of component c) is preferably made of a substantially pure or mixture of different sorbitan fatty acid esters, wherein the sorbitan base body with 1-3 acid radicals of a saturated or unsaturated, straight-chain carboxylic acid and of an even number of 8-20 C-atoms is esterified.

The acid residue of a saturated carboxylic acid having an even number of 8-20 C-atoms, which the sorbitan base body esterified, is preferably straight-chain with 12, 14, 16 and 18 C-atoms, e.g. n-Dodecanoyl, n-Tetradecanoyl, n-Hexadecanoyl or n-Octadecanoyl.

The acid radical of an unsaturated carboxylic acid with an even number of 8-20 C-atoms is preferably straight-chain with 12, 14, 16 and 18 C-atoms.

Preferred sorbitan fatty acid esters are sorbitan-monolaurate, - monopalmitate, - monostearate, - tristearate, - monooleate, - trioleate - sesquioleate.

Particular preference is given to sorbitan mono laurate.

The most preferred sorbitan fatty acid ester is sorbitan mono laurate with a fatty acid composition of lauric acid (C12:0) ≥ 44%; balance primarily myristic (C14:0), palmitic (C16:0) and linolenic (C18:3) acids.

The solubilizer composition may contain additional components, especially preservatives and/or a pH-regulator.

Suitable preservatives are selected from the group consisting of benzoic acid and salts thereof, citric acid and salts thereof, phenoxyethanol, benzyl alcohol, alkyl parabens, preferably ethylparaben, methylparaben and propylparaben, or formaldehyde solution, pentanediol or sorbic acid, silver complexes, and the additional substance classes listed in Annex 6, parts A and B, of the Cosmetics Directive.

PH regulators are preferably used if it is desired to adjust the pH to a skin-friendly pH below pH 7; the pH of the solubilizer composition for cosmetic compositions is typically in the range of pH 3 to 6, more preferably pH 4.0 to 5.5, for oral compositions it is in the range of 4.5 to 7.5, preferably 4.9 to 6.5. Agents or buffers for pH adjustment include citric acid and its salts, tartaric acid and its salts, phosphoric acid and its salts, acetic acid and its salts, hydrochloric acid, sodium hydroxide and sodium bicarbonate. Citric acid is the preferred pH-regulator suitable for pH adjustment of the solubilzer.

### Components to Solubilize:

Perfume oils include mixtures of natural and synthetic origin. Natural odorants are extracts of flowers, stems and leaves, fruit, fruit shells, roots, wood, herbs and grasses, needles and branches, resins and balsams or as well animal raw materials, such as, for example, civet and castoreum, and also synthetic odorant compounds of the ester, ether, aldehyde, ketone, alcohol and hydrocarbon type.

The sparingly soluble perfume oils to be solubilized comprise extracts of flowers such as roses, lilies, lavender, calendula, chamomile, linden blossom, lilly of the valley, jasmine, neroli, passion flower, ylang-ylang; of stems and flowers such as geranium, patchouli, petitgrain; of fruits such as anise, cloves, coriander, caraway, juniper, mango, peach, vanilla; of fruit peels such as bergamot, lemons, oranges; of roots such as mace, angelica, celery, cardamom, costus, iris, calmus; of woods such as pine, sandal, gujak, cedar, rosewood; of herbs and grasses such as tarragon, lemongrass, sage, thyme, rosemary, mint, lemon balm, cinnamon leaves; of needles and twigs such as spruce, fir, pine, mountain pines or of resins and balms such as galbanum, elemi, benzoin, myrrh, olibanum, opoponax.

Fragrances can also include synthetic products of an ester, ether, aldehyde, ketone, alcohols or hydrocarbon.

Fragrance compounds of the ester type are for example benzyl acetate, phenoxyethyl isobutyrate, p-tert.-butylcyclohexyl acetate, linalyl acetate, dimethylbenzylcarbinylacetate, phenylethyl acetate, linalyl benzoate, benzyl formate, ethyl methylphenyl glycinate, allyl cyclohexyl propionate, styrallyl propionate and benzyl salicylate.

Ethers include, for example, benzyl ethyl ether, (dihydro) rose oxide.

Aldehydes can be selected from linear alkanals with 8 to 18 C-atoms, citral, citronellal, citronellyloxyacetaldehyde, cyclamenaldehyde, hydroxycitronellal, lilial and bourgeonal and ketones are ionones, alpha-isomethylionon and methylcedrylketon.

Alcohols include anethole, citronellol, eugenol, geraniol, linalool, phenylethyl alcohol and terpineol.

Often a single fragrance comprises a composition of various specifically combined scent components.

Essential oils mostly used as aroma components are preferably selected from the group consisting of bergamot oil, chamomile oil, rosemary oil, thyme oil, france kinsense oil, juniperberry oil, vetiver oil, lemon oil, lime oil, orange oil, mandarin oil, grapefruit oil, lavender oil, lemongrass oil, linden blossom oil, eucalyptus oil, melissa oil, myrtle oil, mint oil, pine needle oil, rose oil, sage oil, sandal wood oil, tea tree oil, olibanum oil, galbanum oil, labdanum oil, lavandin oil, ylang ylang oil and mixtures thereof.

Preference is also given to using bergamot oil, dihydromyrcenol, lilial, lyral, citronellol, phenylethyl alcohol, α-hexylcinnamaldehyde, geraniol, benzyl acetone, cyclamenaldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, vertofix coeur, evernyl, iraldein gamma, phenylacetic acid, geranyl acetate, benzyl acetate, rose oxide, romillat, irotyl and floramat alone or in mixtures.

Many of the listed essential oils and natural perfume oils are not only fragrances but as well used as flavoring agents for food products such as lemon oil, lime oil, orange oil, mandarin oil, grapefruit oil, eucalyptus oil or peppermint oil.

Another subject of the invention is therefore the use of the solubilizer composition for the solubilization of perfume oils, fragrances, essential oils, dyes, flavorings, nutrients or vitamins. The solubilizer composition has especially shown improved solubilizing properties for perfume oils, fragrances and essential oils.

### Products comprising the solubilizer composition and use of the invention

The inventive solubilizer composition is used in application fields wherein perfume oils, fragrances, flavorings and essential oils are needed to provide a pleasant odor or good taste or mask an unpleasant smell or taste of the products. Generally cosmetic, pharmaceutical, food, personal care and home care formulations often contain these components. Hence another subject of the invention is the use of the inventive solubilizer composition in cosmetic, pharmaceutical, food, personal care and home care formulations. Within the personal care field the solubilization composition has proven itself particularly in the field of oral care and mouth hygiene due to the special tastelessness of the individual components of the solubilizer composition.

Another object of the invention are cosmetic, pharmaceutical, food, personal care or home care products comprising the solubilizer composition according to the invention in amounts of from 0.1 to 30% by weight, preferably 0.5 to 20% by weight, more preferably 0.8 to 10% by weight and in particular 1 to 3 % by weight, based on the finished product.

### Cosmetic Compositions

Cosmetic compositions are to be understood here as meaning all compositions known to the person skilled in the art which are exclusively or primarily intended to be used externally on the human body or in its oral cavity for cleaning, care, protection, maintaining a good condition, perfuming, changing the appearance or for the purposes of influencing body odor. Cosmetic compositions according to the invention contain, in relation to the weight of the composition 0.1 to 30% by weight, preferably 0.5 to 20% by weight, more preferably 0.8 to 10% by weight and in particular 1 to 3 % by weight of the solubilizer composition according to the invention. The cosmetic compositions according to the invention can be in particular formulations for bodycare, facecare, suncare and haircare as well as decorative cosmetics e.g. a body milk, creams, lotions, aftershave lotions, sprayable emulsions, tonics and scented waters, products for eliminating body odor such as deodorants and antiperspirants, make-up removers, conditioners, styling products. The solubilizer composition can also be used in formulations containing further surfactants such as e.g. foam and shower baths, hair shampoos and care rinses.

Depending on the intended application, the cosmetic formulations comprise a series of further auxiliaries and additives, such as, for example, additional surfactants, oil bodies, emulsifiers, pearlescent waxes, consistency regulators, thickeners, superfatting agents, stabilizers, polymers, fats, waxes, lecithins, phospholipids, biogenic active ingredients, UV light protection factors, antioxidants, deodorants, antiperspirants, antidandruff agents, film formers, swelling agents, insect repellents, self-tanning agents, tyrosine inhibitors (depigmentation agents), hydrotropes, solubilizers, dyes.

### Personal Care Products - Oral Care Compositions

Within the personal care composition especially oral care formulations are the preferred field of use due to the advantageous properties regarding the neutral odor and taste of the solubilizer composition. Oral care compositions, such as toothpaste, mouthwash, chewing gum and breath fresheners often include sparingly-soluble components which could easily be dissolved by the inventive solubilizer composition.

Oral care compositions according to the invention contain, in relation to the weight of the composition 0.1 to 30% by weight, preferably 0.5 to 20% by weight, more preferably 0.8 to 10% by weight and in particular 1 to 3 % by weight of the solubilizer composition according to the invention.

Mouthwash, breath fresheners and toothpaste can be aromatized by means of the addition of a suitable conventional aromatizing agent or flavoring agent, very often an aroma of peppermint. Essential oils of spearmint, wintergreen, sassafras, clove, sage, eucalyptus, marjoram, cinnamon, lemon, and orange solubilized in the solubilizer composition of the current invention can likewise be used. The aromatizing agent can be present in an amount from 0.01 to 3 % by weight, preferably 0.05 to 2 % by weight, more preferably 0.1 to 1.5 % by weight with respect to the final oral care composition.

The oral care compositions provided herein may further comprise one or more additional ingredients selected from abrasives, pH modifying agents, surfactants other than a) to c), binding agents, foam modulators, thickening agents, viscosity modifiers, humectants, anti-calculus or tartar control agents, sweeteners, colorants, fragrances, dyes, preservatives, wetting agents, lubricants, remineralisation substances, alcohols, vitamins, water, additional pharmaceutically active ingredients and mixtures of these.

In highly preferred forms of the invention the oral composition may be substantially liquid in character, such as a mouthwash or rinse. In such a preparation the **vehicle** is water or a water-alcohol mixture desirably including a **humectant** selected from sorbitol, glycerine, propylene glycol, xylitol, liquid polyethylene glycol and mixtures thereof. Generally, the weight ratio of water to alcohol is in the range of from about 3:1 to 20:1. The total amount of water or water-alcohol mixture in this type of preparation is typically in the range of from about 70% to about 99.9% by weight of the preparation

Optionally organic **surface-active agents** are used in the compositions of the present invention in addition to the surfactant components of the solubilizer composition to achieve increased prophylactic action, assist in achieving thorough and complete dispersion of the active agents throughout the oral cavity. The organic surface-active material is preferably anionic, nonionic or ampholytic in nature, and it is preferred to employ as the surface-active agent a detersive material which imparts to the composition detersive and foaming properties. Suitable examples of anionic surfactants are water-soluble salts of higher fatty acid monoglyceride monosulfates, such as the sodium salt of the monosulfated monoglyceride of hydrogenated coconut oil fatty acids, higher alkyl sulfates such as sodium lauryl sulfate, alkyl aryl sulfonates such as sodium dodecyl benzene sulfonate, higher alkyl sulfoacetates, higher fatty acid esters of 1,2 dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine which should be substantially free from soap or similar higher fatty acid material. The use of these sarcosinate compounds in the oral composition of the present invention is particularly advantageous since these materials exhibit a prolonged and marked effect in the inhibition of acid formation in the oral cavity due to carbohydrate breakdown in addition to exerting some reduction in the solubility of tooth enamel in acid solutions.

Often the liquid oral composition comprises a **pH-regulator** and a **preservative** in addition to the amounts added by the solubilizer composition. The preservative may be selected from parabens, potassium sorbate, benzyl alcohol, phenoxyethanol, polyaminopropryl biguanide, caprylic acid, sodium benzoate and cetylpyridinium chloride. In some embodiments, the preservative is present at a concentration of from about 0.001 to about 1 weight % (including the amount added by the solubilizer composition) by total weight of the oral care composition.

In certain preferred forms of this invention a **fluoride-providing anti-caries compound** is present in the oral preparation. They are characterized by their ability to release fluoride ions in water and by substantial freedom from reaction with other compounds of the oral preparation. Among these materials are inorganic fluoride salts, such as soluble alkali metal, alkaline earth metal and heavy metal salts, for example, sodium fluoride, potassium fluoride, ammonium fluoride, calcium fluoride, a copper fluoride such as cuprous fluoride, zinc fluoride, a tin fluoride such as stannic fluoride or stannous chlorofluoride, barium fluoride, sodium fluorsilicate, ammonium fluorosilicate, sodium fluorozirconate, sodium monofluoro-phosphate, aluminum mono- and di-flurorophosphate, and fluorinated sodium calcium pyrophosphate. Alkali metal and tin fluorides, such as sodium and stannous fluorides, sodium monofluorophosphate (MFP) and mixtures thereof, are preferred. The amount of the fluoride-providing compound is dependent to some extent upon the type of compound, its solubility, and the type of oral preparation, but it must be a nontoxic amount, generally about 0.01 to about 3.0% in the preparation. In a liquid oral composition the fluoride-providing compound is typically present in an amount sufficient to release up to about 1.0%, preferably about 0.001% to 0.5%, by weight of fluoride ion.

Suitable **sweetening agents** include sucrose, lactose, maltose, sorbitol, xylitol, sodium cyclamate, perillartine, APM (aspartyl phenyl alanine, methyl ester), saccharine and the like. Suitably, flavor and sweetening agents may together comprise from about 0.01% to 5% or more of the preparation.

The oral care compositions, in particular toothpastes, also contain **abrasives.** Suitable abrasives include, without limitation, silica, for example in the form of silica gel, hydrated silica or precipitated silica, alumina, insoluble phosphates, calcium carbonate, resinous abrasives such as urea-formaldehyde condensation products and the like. Among insoluble phosphates useful as abrasives are orthophosphates, polymetaphosphates and pyrophosphates. Illustrative examples are dicalcium orthophosphate dihydrate, calcium pyrophosphate, [beta]-calcium pyrophosphate, tricalcium phosphate, calcium polymetaphosphate and insoluble sodium polymetaphosphate. One or more abrasives are optionally present in the oral care compositions of the present invention in an amount of 1 weight % to 5 weight % by total weight of the composition. The average particle size of an abrasive, if present, is generally 0.1 to 30 µm, and preferably, 5 to 15 µm. The use of these products ensures a low abrasive effect, because it is a case of amorphous solids with a moderate hardness, which at the same time are fully and completely compatible with the fluoride used as the mineralization agent, because they contain no calcic salts, which would cause them to become insoluble and reduce their bioavailability.

The oral care composition comprises at least one **pH modifying agent.** Such agents include acidifying agents to lower pH, basifying agents to raise pH and buffering agents to control pH within a desired range. For example, one or more compounds selected from acidifying, basifying and buffering agents can be included to provide a pH of 3 to 9, or a pH of 2 to 8, 4 to 8, 5 to 7. Any orally acceptable pH modifying agent can be used, including, without limitation, carboxylic, phosphoric and sulfonic acids, acid salts (for example, monosodium citrate, disodium citrate, monosodium malate), alkali metal hydroxides such as sodium hydroxide, carbonates such as sodium carbonate, bicarbonates, borates, silicates, phosphates (for example, monosodium phosphate, trisodium phosphate, pyrophosphate salts) imidazole and the like. One or more pH modifying agents are optionally present in a total amount effective to maintain the composition in an orally acceptable pH range.

Optionally the oral toothpaste composition comprises further **surfactants** in addition to the surfactant components of the solubilizer composition, useful, for example, to provide enhanced stability to the composition and the components contained therein, to aid in cleaning a dental surface through detergent action, and to provide foam upon agitation (for example, during brushing with a dentifrice composition of the invention). Any orally acceptable surfactant, including those which are anionic, nonionic or amphoteric, can be used. Suitable anionic surfactants include, without limitation, water-soluble salts of C8-20 alkyl sulfates, sulfonated monoglycerides of C8-20 fatty acids, sarcosinates, taurates and the like. Suitable nonionic surfactants include, without limitation, poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants, without limitation, derivatives of C8-20 aliphatic secondary and tertiary amines having an anionic group such as carboxylate, sulfate, sulfonate, phosphate or phosphonate. A suitable example is cocoamidopropyl betaine. One or more surfactants are optionally present in a total amount of 0.01 weight % to 10 weight %, for example, from 0.05 weight % to 5 weight % or from 0.1 weight % to 2 weight % by total weight of the composition in addition to the surfactant components of the solubilizer composition.

Organic **thickening agents** can also be incorporated into the oral care compositions, such as sodium carboxymethylcellulose, cellulose ether, Xanthan gum, carrageenan, sodium alginate and carbopols. Inorganic thickening agents, such as silicon oxide thickening agents, sodium aluminium silicates and clays, can also be used to provide the corresponding rheology. The thickening agent can be contained in the formulation in a quantity of 0.5-5 wt. % of the total product.

As a **coloring agent** any of those normally used in the formulation of toothpastes can be used, for example, FCF Brilliant blue, CI.42090 (Kirsch Pharma) The coloring agent can be present in the formulation in an amount lying between 0.001 and 0.005 wt. % with respect to the total. Optionally the oral care composition of the invention comprises a **preservative** in addition to the amount added by the solubilizer composition. The preservative may be selected from parabens, potassium sorbate, benzyl alcohol, phenoxyethanol, polyaminopropryl biguanide, caprylic acid, sodium benzoate and cetylpyridinium chloride. In some embodiments, the preservative is present at a concentration of from about 0.001 to about 1 weight % (including the amount added by the solubilizer composition) by total weight of the oral care composition. For example, as a **sweetener,** sucrose, lactose, maltose, sorbitol, xylitol, sodium cyclamate, perillartine, APM (aspartyl phenyl alanine, methyl ester), saccharine and the like can be used. The sweetener can be present in the formulation in an amount lying between 0.08 and 0.15 wt. % with respect to the total composition. Suitably, flavor and sweetening agents may together comprise from about 0.01% to 5% or more of the preparation.

The **humectant** agent used to prevent desiccation and hardening of the toothpaste is in particular selected from among glycerine, sorbitol, propylene glycol, xylitol and liquid polyethylene glycols, in particular a mixture of sorbitol and glycerine, propylene glycol, xylitol and liquid polyethylene glycol, in particular a mixture of sorbitol, glycerine and xylitol, for example in an amount lying between 1 and 60 wt. % with respect to the total.

As a **lubricant** any of those normally used in toothpaste formulations, for example, dimethycone (polymer of dimethylpolysiloxane), which is a surfactant that contributes to conferring good rheological properties on the toothpaste object of the invention, can be used. The lubricant may be found in the formulation in an amount lying between 0.25 and 0.75 wt. % with respect to the total. As an **opacifier** any of the normal opacifiers may be used, for example titanium dioxide. The opacifier may be present in the formulation in an amount lying between 0.05 and 1 wt. % with respect to the total.

As a **re-mineralising agent** a fluoride source is used, such as sodium fluoride, tin (II) fluoride and sodium monofluorophosphate, as in this way 100% active fluoride is obtained as a re-mineralising agent for the white lesions produced by organic acids arising from bacterial fermentation. The re-mineralising agent can be present in the formulation in an amount lying between 0.2 and 0.4 wt. % with respect to the total.

Typically and furthermore the customary ingredients may be contained, such as anionic **surfactants,** such as e.g. sodium lauryl sulphate, sodium-N-lauryl sarcosinate, sodium lauryl sulfoacetate and sodium alkyl glyceryl ether sulphonate. The surfactant can be contained in the formulation in a quantity of between 0.05 and 5 wt. % with respect to the total in addition to the surfactants used in the solubilizer composition.

The toothpaste proposed by the invention can also contain, if so desired, a **vitamin** selected from the group formed by vitamin A, vitamin B5, vitamin C, vitamin E, and mixtures thereof. If they are used each vitamin can be present in the formulation in a quantity lying between 0.1 and 5 wt. % with respect to the total. These vitamins can be used as they are, in the form of pro-vitamins or in the form of pharmaceutically acceptable salts. Sparingly-watersoluble vitamins are solubilized by the solubilizer composition according to the invention before incorporating into the toothpaste. Vitamin A, which is usually used in the form of palmitate salt, promotes the epithelialisation of oral mucosa and protects the gums. Vitamin B5, more specifically D-pantenol, has a soothing, curative, anti-inflammatory effect, protects the epithelial mucosa, promotes the epithelialisation of injuries and softens scar tissue, and is suitable for the treatment of injuries produced as a consequence of dental extractions, gingivitis, stomatitis, pain produced by putting false teeth in place, ulcers, traumatic lesions of the mucusa and chronic and recurrent cankers. Vitamin C regenerates the epithelium of the oral mucosa, stimulates the synthesis of collagen and the immune system (inflammation mechanism) and increases the capacity for protection of the phagocyte cells against bacteria. Vitamin E, which is usually used in the form of acetate salt, has a calming and anti-inflammatory effect, protects the oral mucosa against lipid peroxidation due to the formation of free radicals and against environmental contaminants (ozone, cigarette smoke. etc.) and favors the healing of injuries. By the incorporation of all or some of the aforementioned vitamins, the invention provides toothpastes that, as well as the aforementioned characteristics, have anti-inflammatory properties and are effective soothing agents, and that increase the protective properties of the membranes of the oral mucosa, and reduce the occurrence of plaque and gingival as well as bacterial contamination.

Additional active ingredients are e.g. **antimicrobial plaque-penetrating agents,** such as beta-naphthol, thymol, chlorothymol and hexylresorcin; or germ-killing compounds such as quaternary ammonium compounds; dental calculus remedies, such as tetrasodium pyrophosphate, GANTREZ-Polymer^{®} S-70, sodium tripolyphosphate and zinc citrate; peroxide compounds, such as hydrogen peroxide and inorganic peroxides.

The presence of potassium ions also provides an effect that alleviates any oversensitivity. The toothpastes can as well contain **water or alcohol** in a proportion of between 1 and 20 wt. % of the total quantity of the agent. In combination with alcohol or instead of the alcohol glycol compounds can also be used such as glycerine, sorbitol or propylene glycol.

### Preparation:

For instance, a mouthrinse or mouthwash may be prepared by mixing ethanol and water with surfactant, humectant, gum or thickener such as sodium carboxymethylcellulose or hydroxyethyl cellulose, and sweetener and adding thereto the solubilized flavor or any other sparingly water-soluble component which is solubilized in the solubilizer composition according to the invention. A toothpaste may be prepared by forming a gel with humectant, gum or thickener such as sodium carboxymethyl cellulose or hydroxyethyl cellulose, and sweetener and adding thereto polishing agent, additional water and the sparingly water-soluble component which is solubilized in the solubilizer composition according to the invention.

### Other products comprising the solubilizer composition

The solubilizer composition may as well be used for food preparations when water-insoluble ingredients such as vitamins, dyes, flavorings or nutrients have to be dissolved in order to achieve a clear transparent product such as for example colored, vitaminized or flavored beverages.

Food products comprising the solubilizer composition according to the invention could be any water containing food composition especially carbonated and still beverages, fruit juice beverages, vegetable juices, fruit juices, fruit juice mixtures, milk, milk beverages, whey and yoghurt beverages, fat spreads, soya milk beverages or high-protein liquid food substitute beverages and fermented milk preparations, yoghurt, drinking yoghurt or cheese preparations, as well as pharmaceutical preparations.

Home care products are often scented in order to mask a negative odor or to achieve a fresh atmosphere after cleaning. Synthetic, natural fragrances or essential oils can easily be dissolved by the inventive solubilizer composition in this kind of products, especially detergents and cleaners. Home care formulations include mainly cleaners, of preference here are in particular products with a low pH for cleaning hard surfaces, such as bath and toilet cleaners and the like, and especially also for cleaning and/or fragrance gels for use in sanitary installations, as well as liquid laundry detergents and fabric softeners.

The use of solubilizers for pharmaceutical applications include the solubilization of water-insoluble drugs or vitamins for oral, cutaneous or parenteral administration. In case of oral administration in form of tablets, dragees, capsules, powders, pills and solutions the solubilization might improve the bioavailability of the solubilized active drug.

### Manufacturing of solubilizer composition and products comprising these compositions

The solubilizer composition is prepared by mixing the respective components under stirring and optionally heating.

For further use of the solubilizer composition one preparation variant is the dissolution of the solubilizer composition in the aqueous phase of the final product, optionally under slight heating and the subsequent release of the sparingly water-soluble component in the aqueous solubilizer solution. The simultaneous dissolution of solubilizer and sparingly water-soluble component in the aqueous phase is likewise possible.

However, preferably the use of the compounds according to the invention as solubilizers is achieved in such a manner, that at first the sparingly water-soluble or sparingly water-dispersible component is dissolved in the mixed solubilizer composition, optionally with heating, under stirring and afterwards this concentrated solution is diluted by mixing with water or aqueous solutions of the final product.

In particular for preparation of the solubilzer composition alkylglucosides are homogenously mixed with citric acid solution and heated to 30 to 60 ° C, preferably 50 to 55°C. Sodium benzoate and acyl glutamic acid compound is added. After complete dissolution of sodium benzoate sorbitan fatty acid ester is added. The solution is stirred until a homogeneous mixture is obtained and cooled to room temperature. If necessary the pH value is adjusted with citric acid solution. The sparingly water-dispersible component can then be dissolved in the mixed solubilizer composition and this solution can be diluted with water or aqueous dispersion or solution of further additives for the requested content of sparingly water-soluble components.

The invention will now be elucidated hereinafter with reference to the examples.

### Examples

### Manufacturing of Solubilizer Compositions

Alkylglucoside (Plantacare 810 UP) was homogenously mixed with citric acid solution (50 % in dem. water) at low stirring speed and heated to 50 ° C. Sodium benzoate and acyl glutamic acid compound (Plantapon ACG) were added at medium stirring speed. After complete dissolution of sodium benzoate sorbitan fatty acid ester (sorbitanmonolaurat) was added. The solution was stirred for approximately 15 minutes until a homogeneous mixture was obtained and cooled to room temperature. If necessary, the pH value was adjusted with citric acid solution to achieve pH 4.9.

The sparingly-watersoluble component (for example perfume oil) was then premixed in the solubilizer composition and this premix was slowly diluted with water for the requested % of active ingredients.

Figures: Solubilization of 1 wt% perfume oil Cotton Touch in aqueous surfactant system of 2wt% active matter each
- Figure 1:: Surfactant compositions comparison a-c (one surfactant component) and inventive d
- Figure 2:: Surfactant compositions comparison a-c (binary surfactant mixtures) and inventive d (ternary mixture)
- Figure 3:: Surfactant compositions comparison (a higher amount of SML, b no SML (comparison) ) and inventive mixture c (adjusted amount of SML)

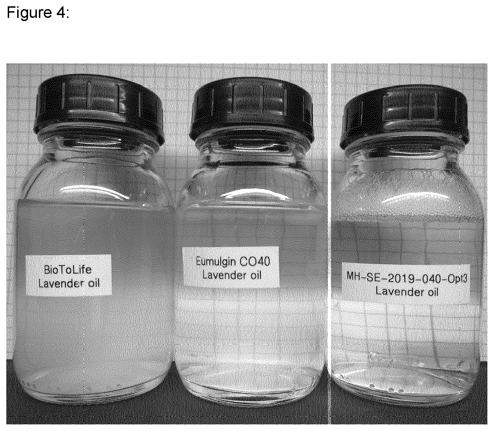

- Figure 4:: Solubilization of Lavender Oil (0.5 wt%) versus solubilizer benchmark (Bio to Life and Eumulgin CO40

Comparative solutions (1 and 2 a to c) were prepared accordingly just omitting the respective surfactant components and adjusting the active matter content. All aqueous surfactant compositions were adjusted to contain 2wt% active matter in the final formulation.

The following compositions have been prepared to compare the solubilization of 1 wt% perfume oil Cotton Touch (Symrise).
1a - APG (Plantacare 818)
1b - ACG (Plantapon ACG 50)
1c - SML (Sorbitan monolaurate)
1d - OPT 3: APG/ACG/SML 2.8:2.0:0.45
2a - APG/ACG 1:1
2b - APG/SML 1:1
2c - ACG/SML 1:1
2d - APG/ACG/SML 2.8:2.0:0.45

Figures 1 a to d and 2 a to d (d is the inventive mixture of APG/ACG/SML) show the improvement of solubilization in view of the pure surfactant components (figure 1) and the binary mixtures (figure 2). Every solution contained 2 wt% active matter based on the composition of a surfactant or surfactant mixture. Only the ternary mixture was able to solubilize 1 wt % of the perfume oil in the aqueous solution. Appearance (transparency) and odour of the solution (figures 1d, 2d) did not change after storage of the compositions comprising OPT3 as solubilizer (for several weeks at room temperature).

The ternary mixture was developed by comparing different amounts of the components APG, ACG and SML.

Figure 3b (OPT2) shows the solubilization of 1 wt% perfume oil Cotton Touch (Symrise) with the binary mixture of APG/ACG 1:1 (same as 2a), while figure 3a shows the same with a ternary mixture comprising a high amount of sorbitan mono laurate. It could be shown that the adjusted amount of SML results in an improvement of solubilization capacity. Appearance (transparency) and odor of the solution did not change after storage - for several weeks at room temperature - of the compositions comprising OPT3 as solubilizer.

**Table 1:**

| | | | **OPT 1** - **comparison** | **OPT 2 - comparison** | **OPT** 3 - **inventive** |
|---|---|---|---|---|---|
| **Ingredient** | **INCI** | | | | |

| | | **Active substance** | **60.1 wt%** | **55.0 wt%** | **57.6 wt%** |
|---|---|---|---|---|---|
| | | **APG/ACG/SML-ratio** | **56.5/30/13.5** | **50/50/0** | **53/40/7** |
| | | | **wt [%] active matter** | **wt [%] active matter** | **wt [%] active matter** |
| Plantacare 810 UP | Caprylyl / Capryl Glucoside | | 31.46 | 24.68 | 28.08 |
| Citric Acid Solution | Citric Acid | | 3.82 | 5.16 | 4.48 |
| Sodium Benzoate | Sodium Benzoate | | 0.50 | 0.50 | 0.50 |
| Sorbitanmonolaurate | Fatty acid C8-18/18:1 sorbitan mono ester | | 7.53 | 0 | 3.77 |
| Plantapon ACG 50 | Sodium Cocoyl Glutamate | | 16.80 | 24.68 | 20.74 |
| Aqua dem. | | | 39.89 | 44.98 | 42.43 |

**Table 2: Solubilization of different Perfume Oils - comparison with benchmark "Bio to Life" (BASF Asia) and standard EO-containing solubilizer Eumulgin CO40 (BASF)**

| The following amounts of solubilizer were necessary to achieve clear solutions of 1 wt% perfume oil (Tea tree oil, Cotton Touch) or 0.5 wt% (Lavender oil, see Figure 3) in water. | | | | |
|---|---|---|---|---|
| **Oil + x% solubilizer Add 100 water** | **wt% oil** | **wt% solubilizer Bio ToLife** | **wt% solubilizer Eumulgin CO40** | **wt% active matter solubilizer (inventive)*OPT3** |
| Tea tree oil (Bergland Pharma) | 1 | 9 | 6 | 2 |
| Perfume oil Cotton Touch | 1 | 4 | 3 | 1 |
| Lavender oil (Düllberg) | 0,5 | 5 | 2 | 3 |

| | | | | |
|---|---|---|---|---|
| * wt % solubilizer needed to solubilize the oil | | | | |

### Oral Care Compositions comprising the solubilizer composition

### Mouthwash solution:

| **Phase** | **Product** | Amounts in weight (g) | | |
|---|---|---|---|---|
| **A** | Sorbic acid | | 0.20 | |
| | Benzoic acid | 0.20 | | 0.20 |
| | Menthol | | 0.20 | |
| | Peppermint oil (Oleum Menthae pip. - dopp. rectific., Caesar & Loretz) | 0.20 | | 0.20 |
| **B** | **OPT 3 (active matter: 57,57%)** | | 2.45 | 2.45 |
| | **Eumulgin CO 40** | 1.40 | | |
| | Ethanol | 7.00 | 7.00 | 7.00 |
| **C** | Glycerin | 12.00 | 12.00 | 12.00 |
| | Water | ad 100.0 | ad 100.0 | ad 100.0 |
| | | | | |
| | pH-value | 5.00 | 5.04 | 5.00 |
| | appearance: | clear | clear | clear |
| D | NaOH (10%) to adjust pH | 0.3 | 0.35 | 0.3 |

The mouthwash is prepared by mixing the components at room temperature. At first benzoic (sorbic acid) and peppermint oil or menthol is thoroughly mixed with the solubilizer, afterwards ethanol is added, finally the glycerol/water mixture is added and mixed to achieve a clear solution.

## Claims

1. Solubilizer composition which is free from ethoxylated emulsifiers comprising
a) 20 to 40 wt % of alkyl glucosides,
b) 15 to 30 wt % of acyl glutamic acid compounds,
c) 2 to 6 wt % of a sorbitan fatty acid ester and
optionally 10 to 63 wt % of water, all wt % based on the weight of the composition and amounts given in wt % of active matter.

2. Solubilizer composition as claimed in claim 1, wherein component a) is an alkyl oligoglucoside according to formula (I)
R¹O-[G]p (I),
in which R¹ is an alkyl and/or alkenyl radical with 8 to 18 carbon atoms, G is glucose and p is numbers between 1 and 10.

3. Solubilizer composition as claimed in claim 1 and/or 2, wherein component b) is an acylglutamic acid compound according to formula (II)
M₁OOC-CH₂-CH₂-CH(NH-CO-R²)-COOM₂ (II)
in which the radical R² is a linear or branched alkyl or alkenyl radical with 7 to 19 carbon atoms and the radicals M₁ and M₂ - independently of one another - are selected from the group H, Li, Na, K, Ca/2, Mg/2, ammonium and alkanolamines, preferably sodium cocoyl glutamate.

4. Solubilizer composition as claimed in one of claims 1 to 3 wherein component c) is a sorbitan fatty acid ester selected from the group consisting of sorbitan-monolaurate, - monopalmitate, - monostearate, - tristearate, - monooleate, - trioleate and sorbitan sesquioleate.

5. Solubilizer composition as claimed in one of claims 1 to 4, wherein the sorbitan fatty acid ester is sorbitan mono laurate.

6. Solubilizer composition as claimed in one of claims 1 to 5 comprising a pH-regulator and/or a preservative as additional components.

7. Use of the solubilizer composition according to any one of claims 1 to 6 in cosmetic, pharmaceutical, food, personal care or home care products.

8. Use of the solubilizer composition according to any one of claims 1 to 6 for the solubilization of perfume oils, fragrances, essential oils, dyes, flavorings, nutrients or vitamins.

9. Oral care composition comprising 0.1 to 30 wt% of the solubilizer composition according to one of claims 1 to 6 based on the weight of the oral care composition.

## Patentansprüche

1. Lösungsvermittlerzusammensetzung, die frei von ethoxylierten Emulgatoren ist, umfassend
a) 20 bis 40 Gew.-% Alkylglucoside,
b) 15 bis 30 Gew.-% Acylglutaminsäureverbindungen,
c) 2 bis 6 Gew.-% eines Sorbitanfettsäureesters und
gegebenenfalls 10 bis 63 Gew.-% Wasser, wobei alle Gew.-%-Angaben auf das Gewicht der Zusammensetzung bezogen sind und Mengen in Gew.-% Wirkstoff angegeben sind.

2. Lösungsvermittlerzusammensetzung nach Anspruch 1, wobei es sich bei Komponente a) um ein Alkyloligoglucosid gemäß Formel (I) handelt
R¹O-[G]p (I),
worin R¹ für einen Alkyl- und/oder Alkenylrest mit 8 bis 18 Kohlenstoffatomen steht, G für Glucose steht und p für Zahlen zwischen 1 und 10 steht.

3. Lösungsvermittlerzusammensetzung nach Anspruch 1 und/oder 2, wobei es sich bei Komponente b) um eine Acylglutaminsäureverbindung gemäß Formel (II) handelt
M₁OOC-CH₂-CH₂-CH(NH-CO-R²)-COOM₂ (II)
worin der Rest R² für einen linearen oder verzweigten Alkyl- oder Alkenylrest mit 7 bis 19 Kohlenstoffatomen steht und die Reste M₁ und M₂ - unabhängig voneinander - aus der Gruppe H, Li, Na, K, Ca/2, Mg/2, Ammonium und Alkanolaminen, vorzugsweise Natriumcocoylglutamat, ausgewählt sind.

4. Lösungsvermittlerzusammensetzung nach einem der Ansprüche 1 bis 3, wobei es sich bei Komponente c) um einen Sorbitanfettsäureester handelt, der aus der Gruppe bestehend aus Sorbitanmonolaurat, -monopalmitat, - monostearat, -tristearat, -monooleat, -trioleat und Sorbitansesquioleat ausgewählt ist.

5. Lösungsvermittlerzusammensetzung nach einem der Ansprüche 1 bis 4, wobei es sich bei dem Sorbitanfettsäureester um Sorbitanmonolaurat handelt.

6. Lösungsvermittlerzusammensetzung nach einem der Ansprüche 1 bis 5, umfassend als zusätzliche Komponenten einen pH-Regler und/oder ein Konservierungsmittel.

7. Verwendung der Lösungsvermittlerzusammensetzung gemäß einem der Ansprüche 1 bis 6 in kosmetischen, pharmazeutischen, Lebensmittel-, Körperpflege- oder Haushaltspflegeprodukten.

8. Verwendung der Lösungsvermittlerzusammensetzung gemäß einem der Ansprüche 1 bis 6 zur Solubilisierung von Parfümölen, Duftstoffen, ätherischen Ölen, Farbstoffen, Geschmacksstoffen, Nährstoffen oder Vitaminen.

9. Mundpflegezusammensetzung, umfassend 0,1 bis 30 Gew.-% der Lösungsvermittlerzusammensetzung gemäß einem der Ansprüche 1 bis 6, bezogen auf das Gewicht der Mundpflegezusammensetzung.

## Revendications

1. Composition de solubilisant qui est exempte d'émulsifiants éthoxylés comprenant
a) 20 à 40 % en poids d'alkylglucosides
b) 15 à 30 % en poids de composés d'acide acylglutamique,
c) 2 à 6 % en poids d'un ester d'acide gras de sorbitane et
éventuellement 10 à 63 % en poids d'eau, tous les % en poids étant basés sur le poids de la composition et les quantités indiquées en % en poids de matière active.

2. Composition de solubilisant selon la revendication 1, dans laquelle le composant a) est un alkyoligoglucoside de formule (I)
R¹O-[G]p (I),
dans laquelle R¹ est un radical alkyle et/ou alcényle ayant 8 à 18 atomes de carbone, G est glucose et p est des nombres entre 1 et 10.

3. Composition de solubilisant selon la revendication 1 et/ou 2, dans laquelle le composant b) est un composé d'acide acylglutamique de formule (II)
M₁OOC-CH₂-CH₂-CH(NH-CO-R²)-COOM₂ (II)
dans laquelle le radical R² est un radical alkyle ou alcényle, linéaire ou ramifié, ayant 7 à 19 atomes de carbone et les radicaux M₁ et M₂, indépendamment l'un de l'autre, sont choisis dans le groupe H, Li, Na, K, Ca/2, Mg/2, ammonium et alcanolamines, de préférence cocoylglutamate de sodium.

4. Composition de solubilisant selon l'une des revendications 1 à 3, dans laquelle le composant c) est un ester d'acide gras de sorbitane choisi dans le groupe constitué par le monolaurate, monopalmitate, monostéarate, tristéarate, monooléate, trioléate de sorbitane et le sesquioléate de sorbitane.

5. Composition de solubilisant selon l'une des revendications 1 à 4, dans laquelle l'ester d'acide gras de sorbitane est le monolaurate de sorbitane.

6. Composition de solubilisant selon l'une quelconque des revendications 1 à 5, contenant comme composants supplémentaires un régulateur de pH et/ou un conservateur.

7. Utilisation de la composition de solubilisant selon l'une quelconque des revendications 1 à 6 dans des produits cosmétiques, pharmaceutiques, alimentaires, de soins personnels ou d'entretien ménager.

8. Utilisation de la composition de solubilisant selon l'une quelconque des revendications 1 à 6 pour la solubilisation d'huiles parfumées, de fragrances, d'huiles essentielles, de colorants, d'arômes, de nutriments ou de vitamines.

9. Composition de soins buccaux comprenant 0,1 à 30 % en poids de la composition de solubilisant selon l'une quelconque des revendications 1 à 6, sur la base du poids de la composition de soins buccaux.
